# EUROPEAN PATENT APPLICATION

(11) **EP 1 903 462 A2**
(43) Date of publication of application: **26.03.2008**
(21) Application number: 07252580.1
(22) Date of filing: 26.06.2007
(51) Int. Cl.: G06F 19/00

(54) **A clinical trial data processing and monitoring system.**

(30) Priority: 24.08.2006 US 823417 P; 07.06.2007 US 759270
(71) Applicant: Siemens Medical Solutions USA, Inc., Malvern, PA 19355-1406 (US); Siemens Aktiengesellschaft, 80333 Munich (DE)
(72) Inventor: Zahlmann, Gudrun Wirkner, 92318 Neumarkt (DE); Wronka, Andrew, Port Monmouth, New Jersey 07758 (US); Schmidt, Markus, 90425 Nuernberg (DE); Brandon, Paul, Sanatoga, Pennsylvania 19464 (US)
(74) Representative: Clarke, Alison Clare

(57) **Abstract**

A patient image data processing system comprises a first validation processor for parsing a message conveying patient medical image data from an image provider to identify image metadata indicating first characteristics of the image. The first validation processor performs a first comparison by comparing the metadata with configuration data indicating predetermined characteristics of images required for a particular use and provides first acceptability data indicating the image is unacceptable for the use in response to an unsuccessful first comparison. A second validation processor parses imaging metadata representing the image to identify image metadata indicating second characteristics of the image. The second validation processor performs a second comparison by comparing header data with configuration data indicating predetermined characteristics of images required for a particular use and provides second acceptability data indicating the image is unacceptable for the use in response to an unsuccessful second comparison. A data processor generates performance data using the first and second acceptability data indicating quality of images provided by the image provider.

## Description

This is a non-provisional application of provisional application serial No. 60/823,417 by P. Brandon et al. filed August 24, 2006.

### Field of the Invention

This invention concerns a patient image data processing system, for validating that image data is suitable for use in a clinical trial, for example, by examining image metadata and image message header data and generating performance data identifying quality of images provided by an image provider.

### Background of the Invention

Clinical trials are often required for getting a new drug approved by a regulatory agency like the FDA (Federal Drug Administration). The effect of a new therapeutic or diagnostic test on humans needs to be proven by following a clearly defined test procedure that is described in detail in a clinical trial protocol. Clinical trials are conducted by clinicians using their patients in hospitals, physician offices or comparable facilities. It is done fulfilling a contract with a trial sponsor (such as a pharmaceutical company). Sponsors often outsource the management of the clinical trial to so called CROs (Contract Research Organisations). In the case of clinical trials that rely on medical imaging in the trial results specialized imaging CROs fulfil this task. Management of the CROs by the sponsor or the clinical sites by the CRO are often supported by technical systems (excel spreadsheet, trial management systems etc.) that need manual input and assessment by clinical trial collaborators regarding quality and performance of the data providers. CRO information systems have limited integration and interoperability with electronic data capture or clinical data management systems and typically have no interoperability with sites that are providing imaging data. The independence of a CRO as a separate entity from a trial sponsor further limits level of coordination of processes or implementation of equipment solutions. The integration and coordination capabilities of known systems are particularly limited in processing imaging data in clinical trials. A system according to invention principles addresses these deficiencies and associated problems.

### Summary of the Invention

A system compares clinical trial protocol data in a configuration file with medical image metadata files and image header data to derive quality assurance and control process data to monitor performance of clinical sites and imaging research organizations for process improvement. A patient image data processing system comprises a first validation processor for parsing a message conveying patient medical image data from an image provider to identify image metadata indicating first characteristics of the image. The first validation processor performs a first comparison by comparing the metadata with configuration data indicating predetermined characteristics of images required for a particular use and provides first acceptability data indicating the image is unacceptable for the use in response to an unsuccessful first comparison. A second validation processor parses imaging metadata representing the image to identify image metadata indicating second characteristics of the image. The second validation processor performs a second comparison by comparing header data with configuration data indicating predetermined characteristics of images required for a particular use and provides second acceptability data indicating the image is unacceptable for the use in response to an unsuccessful second comparison. A data processor generates performance data using the first and second acceptability data indicating quality of images provided by the image provider.

### Brief Description of the Drawing

Figure 1 shows a patient image data processing system for monitoring performance of clinical sites and imaging research organizations, according to invention principles.
Figure 2 shows a further patient image data processing system for monitoring quality and performance of clinical sites and imaging research organizations, according to invention principles.

### Detailed Description of the Invention

Imaging data used herein comprises image representative data and correlated metadata (e.g. DICOM images that contain pixel data and metadata in the form of a DICOM header. The DICOM -Digital Imaging and Communications in Medicine - standard developed by the American College of Radiology Manufacturers Association defines connectivity and communication protocols of medical imaging devices.). A processor, as used herein, operates under the control of an executable application to (a) receive information from an input information device, (b) process the information by manipulating, analyzing, modifying, converting and/or transmitting the information, and/or (c) route the information to an output information device. A processor may use, or comprise the capabilities of, a controller or microprocessor, for example. The processor may operate with a display processor or generator. A display processor or generator is a known element for generating signals representing display images or portions thereof. A processor and a display processor comprise any combination of, hardware, firmware, and/or software.

An executable application, as used herein, comprises code or machine readable instructions for conditioning a processor to implement predetermined functions, such as those of an operating system, a context acquisition system or other information processing system, for example, in response to user command or input. An executable procedure is a segment of code or machine readable instruction, subroutine, or other distinct section of code or portion of an executable application for performing one or more particular processes. These processes may include receiving input data and/or parameters, performing operations on received input data and/or performing functions in response to received input parameters, and providing resulting output data and/or parameters.

A user interface (UI), as used herein, comprises one or more display images, generated by a display processor and enabling user interaction with a processor or other device and associated data acquisition and processing functions. The UI also includes an executable procedure or executable application. The executable procedure or executable application conditions the display processor to generate signals representing the UI display images. These signals are supplied to a display device which displays the image for viewing by the user. The executable procedure or executable application further receives signals from user input devices, such as a keyboard, mouse, light pen, touch screen or any other means allowing a user to provide data to a processor. The processor, under control of an executable procedure or executable application manipulates the UI display images in response to the signals received from the input devices. In this way, the user interacts with a display image using the input devices, enabling user interaction with the processor or other device. The functions and process steps herein may be performed automatically or wholly or partially in response to user command. An activity (including a step) performed automatically is performed in response to executable instruction or device operation without user direct initiation of the activity. Workflow comprises a sequence of tasks performed by a device or worker or both. An object or data object comprises a grouping of data, executable instructions or a combination of both or an executable procedure. A document or record comprises a compilation of data in electronic or paper form.

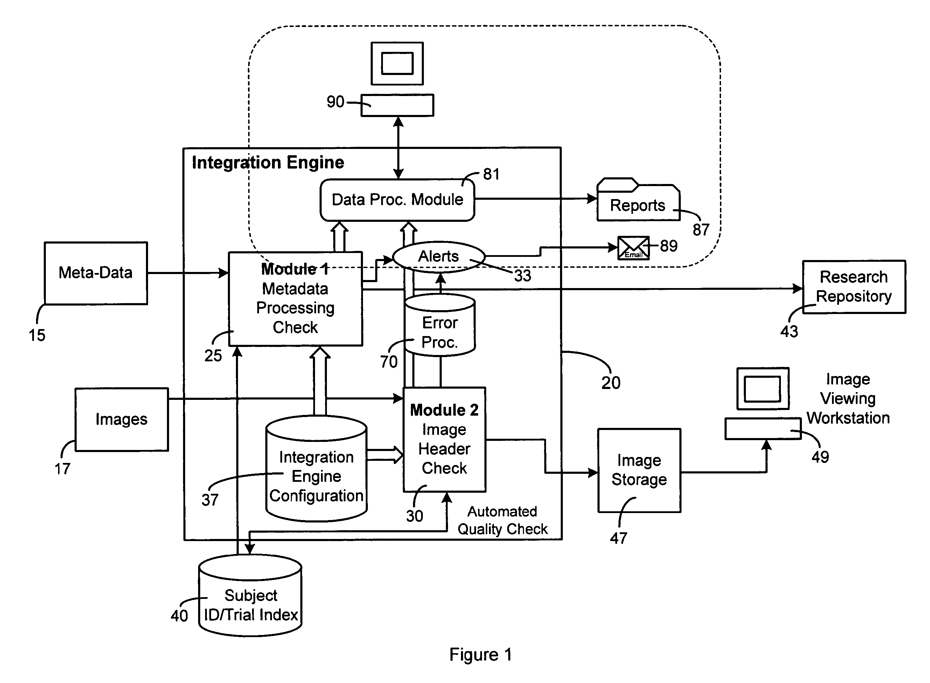

Figure 1 shows patient image data processing system 10. The operation of a clinical trial is guided by a structured trial protocol which prescribes the order of steps to be performed and the data to be collected. Images are increasingly included in trials, however, whereas physical measurements such as blood pressure can be easily checked, images present an added complexity both for operation of a trial and for quality checks. The process of generating images is complex and requires highly trained personnel that are accustomed to being innovative in determining the best image equipment parameters to generate an appropriate image quality to enable a radiologist to make qualified diagnostic decisions. However, a structured clinical trial protocol requires use of specific parameters and constraints to ensure that images taken over a period of time may be compared in order to assess an impact of a new drug. In known systems a technologist acquires an image and a radiologist assesses the image based upon an understanding of the trial procedures. However, over the lengthy period of a clinical trial (e.g., several years) errors commonly occur.

In known systems, a sponsor of a clinical trial typically receives an assessment of an image for use in the trial from an image service provider and has little or no access to the image itself and little opportunity to perform quality checks on the image. Image data used in clinical trials is usually not handled by pharmaceutical companies and therefore is excluded from automated data management functions. In the case where an image is received, the sponsor employs a manual process for performing image quality checks. Errors in the image, such as the imaging of a wrong body part or incorrect parameters being associated with the image, are found by expert reviewers by examining the image and associated metadata file(s) data. During image acquisition, a technologist configures imaging equipment to obtain an image of a desired body part with desired parameters as specified in the trial protocol. The imaging equipment produces an image or image series along with metadata describing the image, such as the body part, the number of images in the series and contrast media used, which is included in image (e.g., DICOM compatible) metadata. In addition, metadata describing an image, such as a radiologist assessment, is produced and stored in a radiology or other departmental information system supporting imaging department operation. Image representative data and correlated metadata is typically stored in a picture archiving and communications systems (PACS).

In system 10 of Figure 1, in response to image representative data and associated metadata being entered into image storage and radiology systems, transaction messages, e.g., HL7 (HealthLevel7) compatible messages containing metadata 15 and imaging data 17 containing image representative data and header information are produced and communicated to integration engine 20 in system 10. System 10 assists in a clinical trial process by providing automated quality checks of clinical trial images to ensure a correct image is acquired using parameters specified in a trial protocol and provides statistics indicating quality of images and related data received from different image service providers. Data derived from quality assurance and control process checks performed by integration engine 20 is processed by data processor (module) 81 to provide information (e.g., via workstation 90) about clinical sites and imaging research organizations that is used by integration engine 20 in process improvement. During automated quality checks of images 17 and associated metadata 15, information about an image and the process that generated it is obtained. Integration engine 20 identifies a source of an image by use of a map associating a clinical site and a connection destination through which image data is received. The map is derived using information available from calls to initiate execution of applications via an Application Programming Interface (API) of integration engine 20 as well as from data indicating access to data content of metadata transactions and imaging metadata. This information includes data identifying, a clinical site providing a digital image, a modality (imaging) device that generated an image, a parameter used to generate an image, a digitization procedure used in generating image data and an initial quality of an image (i.e. was it rejected or accepted as a result of an automated quality control (QC) check) and a reason an image failed an automated QC check.

Additional information is also derived from processing of an image including data identifying clinical sites that rely on film and provide a metadata transaction describing an image, a percentage of images of a given site or modality device that pass an automated quality check versus those images that fail checks and an error rate per image for a clinical site or modality device. The information provided by unit 81 from quality assurance processing indicates quality of images and information received from an image provider based upon a number of images and associated metadata that are accepted or rejected in a quality control process. This information is summarized in managerial reports 87 providing statistics reflecting the performance of image suppliers.

System 10 collects and summarizes available data about the processing of images for a clinical trial to support assessment and management of performance of clinical sites and imaging CROs. System 10 includes configuration unit 37 incorporating information from a clinical trial protocol identifying data to gather about the processing of images for each clinical trial and indicating predetermined characteristics of images conforming to clinical trial requirements during an analysis of the trial protocol and image and metadata specifications. Data processor 81 acquires, collates and stores information in integration engine 20 in response to processing individual image headers or metadata files and system 10 provides reports 87 that support analysing performance trends for a clinical trial by comparing individual clinical trial site performance for the duration of a trial. System 10 also generates alert messages 33 for communication (e.g., via email link 89) to a user indicating an error has occurred that can impact the timeline of a trial, indicating the need for manual review and corrective action.

The details of a trial protocol vary by trial, consequently configuration unit 37 provides an interface enabling automatic configuration (or user manual configuration via workstation 90) of data that needs to be acquired for an individual metadata transaction and from metadata of an individual imaging data set. Engine 20 identifies the data indicated as needing to be acquired, in processing of an image and associates it to parameters that are used to produce reports 87. For example, engine 20 identifies that for trial ABC, any out of range data element corresponds to a violation of a trial protocol while an absence of a data element corresponds to a quality issue to be indicated to a user.

Details of a trial protocol vary by trial and configuration unit 37 enables a user to configure integration engine 20 to identify the details that need to be captured for each metadata transaction and each DICOM header of an individual trial. The configuration of integration engine 20 involves making reference information for a quality check procedure visible. Thereby a quality check measures incoming data against reference values (thresholds, limits, etc.) that are determined by an image study trial protocol. If the study protocol is provided in electronic form in a standardized format (like CDSIC) integration engine 20 extracts the reference values and presents them in a graphical form to a user configuring integration engine 20. and the system also enables manual addition and entry of additional values. The system also interfaces to various data sources individually containing various data models. Integration engine 20 creates an image view for a user listing elements of the data models of the data sources and enabling a user to select single fields as input fields for a quality check

The configuration information identifies data derived from processing of an image and associates it with parameters that are used to produce management reports 87. Configuration unit 37 enables user or automated data entry of trial protocol operational parameters and thresholds used to generate alerts, including, timelines with defined intervals for image acquisition per patient (e.g., time between image study imaging visits), a number of images per study and a number of images per patient as well as contract and performance milestones such as a deadline for recruiting patients for a trial, for example. Unit 37 also enables user or automated data entry of data items to be acquired from imaging metadata or metadata such as data indicating an imaging modality device (e.g., CT scan, MR, Ultrasound, X-ray) used, image sequence, location of the patient, imaging device coils being used, etc. Unit 37 further enables user or automated data entry of data indicating required imaging procedures per patient per examination (e.g., imaging of thorax), imaging modality device settings to be used (e.g., image to be obtained from an MRI device with specific configuration parameters set). System 10 enables a user to indicate data to be acquired using configuration unit 37 and maps acquired data to labels that represent attributes of a data source such as the name of an image provider. For example, data indicating a source connection or application name that received data is acquired and a map indicates an associated source name that represents image provider XYZ.

In response to processing an image header or metadata transaction for quality assurance monitoring, integration engine 20 initiates execution of units 25 and 30. Units 25 and 30 acquire and collate data items that are identified by configuration unit 37 by calling Application Programming Interfaces (APIs) initiated by integration engine 20 to provide access to data items in imaging metadata and metadata in transaction messages (e.g., indicating type of imaging modality, slice thickness, etc.) using stored integration engine 20 parameters (e.g., source connection name) or data identifying errors. Units 25 and 30 acquire and record data items including, a date and time stamp of the processing event obtained from a system clock or from integration engine 20, a name of an image provider derived using a name of a source connection within integration engine 20 that received the image and metadata, a Subject identifier obtained from content of the metadata or the imaging metadata and parameters copied from the imaging metadata or from within the metadata (e.g. modality producing the image). Units 25 and 30 also acquire and record data items including parameters used to capture an image, image accession number, and number and type of errors encountered in associated image headers or metadata transactions during processing obtained by integration engine 20 as it performs quality control (QC) checks and whether an image passed or failed a QC check.

Integration engine 20 produces reports 87 using the acquired data stored in a database in engine 20. Reports 87 summarize the data that has been collected during processing of the images and provide information that supports actions including those described below. Additional reports 87 provide insight into performance of clinical trial image providers or issues related to a clinical trial protocol. Integration engine 20 identifies high quality and high performance clinical sites and imaging CROs by providing data indicating types and number of errors encountered during performing quality assurance checks per image provider for a specified period of time (e.g., a previous week, previous month, previous year, etc.). Reports 87 list date and time of each processing event which failed a quality assurance check and an associated indication of the reason for failure (i.e., describing an error) that occurred. In addition, a summary report 87 is generated that lists a total number of errors per image provider for a specified period of time and image providers are identified that are providing images with the lowest number of errors.

Integration engine 20 identifies under-performing image providers and clinical sites and imaging CROs by providing data in reports 87 indicating a number of images submitted by a clinical site or other image provider along with an indication of status associated with individual performance measurements specified in configuration unit 37. For example, reports 87 list individual image providers, the number of images per recruited trial patient, the number of patients for which a record appears in the database for the given period of time, the total number of images processed successfully during a predetermined period of time and a calculated average number of images per patient.

Integration engine 20 further identifies difficulties in quality related to a clinical trial protocol by providing data in reports 87 concerning quality issues related to the definition of the protocol. These are obtained by listing errors of a selected type (the types that can be selected are related to clinical trial protocol parameters entered in configuration unit 37) for each image provider. For example, a clinical trial protocol requires that images are generated by a CT scanner and reports 87 includes a generated list indicating failure events occurring due to an incorrect imaging modality device being listed in the imaging metadata. Integration engine 20 also intermittently reviews the acquired collated data in the database in unit 20 and compares trial operational parameters with information collected for a clinical site or CRO. The operational parameters (e.g., number of images per study, number of images per patient, etc.) are obtained from a trial protocol and recorded in configuration unit 37. Integration engine 20 reviews its internal database and compares planned progress expected from a clinical site or CRO with data obtained and stored in the database to determine if trial progress falls within predetermined acceptable trial parameters. If trial progress does not fall within an acceptable progress range, an alert message 33 is generated and emailed by unit 89 to a healthcare worker performing a particular role (e.g., a trial Coordinator) so that corrective action (e.g., contacting a clinical site or CRO) is taken to avoid impact to a trial schedule.

In an example of operation, CRO 123 is responsible for submitting a minimum number of image sets per month per trial subject. Data identifying images provided for each trial subject assigned to CRO 123 for the previous 30 days is obtained from the unit 20 database by reading entries submitted by CRO 123 that have a date and time stamp within a 30 day period and that have a flag indicating successful quality assurance processing. The number of images per subject is determined by counting the number of entries for each subject identifier. An alert message 33 is generated for subjects that do not have a minimum number of image sets successfully passing integration engine 20 quality assurance performance checks. Email unit 89 communicates generated alert messages 33 using an API initiated by integration engine 20 which provides unit 89 with data identifying a message recipient, a sender and email content and records tracking data concerning the communication of the generated alert message in an audit log.

System 10 enables a trial sponsor to accept images from clinical sites and imaging CROs and to perform quality assurance checks and control process improvement and monitoring. The quality assurance checks provide information about the clinical sites and imaging CROs involved. The quality assurance information is summarized in managerial reports 87 showing statistics indicating, which clinical site provides digital images, which rely on film, which parameters are used to generate an image, data identifying a digitization procedure used and readout results of image content, for example. In addition, as result of the system 10 QC (Quality Control) process, system 10 generates statistics in reports 87 data indicating, how often acceptable and adequate quality image data is provided by an imaging CRO and how often unacceptable quality image data was provided as well as indicating which clinical site is responsible failing to provide adequate quality images and an associated reason. System 10 compares statistics in reports 87 with a clinical trial protocol definition in unit 37 and determines if there is a violation of the trial protocol or if there is a violation ambiguity resulting from lack of fully defined clinical trial parameters, for example.

System 10 provides data and data summaries in reports 87 using a hierarchical process that guides managing clinical sites and imaging CROs and identifies, high quality, high performing and under-performing clinical sites and imaging CROs as well as difficulties in quality related to a clinical trial protocol. System 10 further compares performance over a timeline of a trial and generates alert messages 33 in response to identifying trial delays. System 10 advantageously provides online and timely control of quality relevant parameters for individual sites involved in a clinical trial and individual imaging CROs. System 10 provides an automatic alerting service indicating quality violations and time delays and an automatic procedure based on incoming data independent of manual data entry and interaction. System 10 further provides automated clinical trial data processing with minimal manual or programming staff involvement and enables a trial sponsor to accept images from clinical sites and imaging CROs and manage quality assurance and process improvement. For this purpose, system 10 includes a configuration interface enabling identification of metadata to be acquired concerning processing of images for each clinical trial. Interface engine 20 acquires quality information in response to processing individual image headers and metadata files. A report generator in engine 20 generates reports 87 that support analysing performance trends of a trial by comparing trial site performance over the duration of a trial and system 10 produces alert messages 33 when a trial operation delay occurs.

System 10 provides a User Interface, e.g., on workstation 49, for the creation, management, and maintenance of a clinical trial definition that provides intelligence and instruction enabling clinical trial quality control to be automated. The definition includes both instance level criteria and trial context criteria. For a specific trial definition, the instance level criteria are used to check that input data (e.g., image representative data and associated metadata) values satisfy trial requirements for a procedure. Clinical trial context criteria are used by the system to validate appropriateness of a procedure for a particular patient in the context of where the patient is currently in a trial timeline.

System 10 integration engine 20 includes a first validation processor (metadata processing module) 25 for performing an automated check of metadata of an image and a second validation processor (image header data processor module) 30 for performing an automated check of the imaging metadata. First validation processor 25 parses a message conveying patient medical image data from an image provider (e.g., a clinical trial participant) to identify image metadata indicating first characteristics of the image and performs a first comparison by comparing the metadata with configuration data (e.g., also metadata) indicating predetermined characteristics of images required for a particular use (conforming to clinical trial requirements) and provides first acceptability data indicating the image is unacceptable for the use in response to an unsuccessful first comparison Second validation processor 30 parses imaging metadata to identify image metadata indicating second characteristics of the image. Second validation processor 30 performs a second comparison by comparing header data with configuration data indicating predetermined characteristics of images required for a particular use and provides second acceptability data indicating the image is unacceptable for the use in response to an unsuccessful second comparison. Data processor 81 indicates the image is acceptable for use in a clinical trial in response to successful first and second comparisons and generates performance data using the first and second acceptability data indicating quality of images provided by the image provider. Data processor 81 generates the performance data comprising statistical information indicating relative proportion of acceptable and unacceptable images provided by the image provider using the first and/or second acceptability data and data indicating a number of acceptable images provided by the image provider, within a particular time duration The performed comparisons may include exact match, range comparison, comparison with predetermined acceptable values, comparison to see if below or above a predetermined threshold, a number comparison, a text comparison or a comparison with a value computed by the system, for example. Error processor 70, in response to unsuccessful first and second comparisons, automatically (or in response to user command) initiates generation of alert message 33 to a user indicating an image is unacceptable and automatically (or in response to user command) routes image data to a storage location for further manual or automatic processing.

Information from a clinical trial protocol is used to populate configuration data in configuration unit 37 with data indicating predetermined characteristics of images conforming to clinical trial requirements during an analysis of the trial protocol and image and metadata specifications. Information such as data identifying body parts to be imaged and parameters of the image capture (e.g., MRI image with proper sequence, gradient, correct coil) is expressed in configuration and edit checks of integration engine 20 operation. The checks that are performed differ from trial to trial and are based upon the trial protocol. During processing, integration engine 20 queries external source of information 40 to associate a patient identifier supplied with image and metadata 15, 17 to a subject ID acquired from source of information 40 used in the trial. This query is also used to determine if the patient is in an active study and in which study. Integration engine 20 establishes communication connections to data sources in image repository (or file server) 47 and Research repository 43 in parsing and formatting transactions.

Integration engine 20 receives metadata 15 in the form of a message or transaction in HL7 format, for example. The information entered into configuration unit 37 from the trial protocol is checked against the data in a received HL7 transaction. Configuration unit 37 contains information for Study XYZ indicating that MRI images of a patient head are included in the trial, for example. Configuration unit 37 information also indicates other mandatory criteria for received metadata 15. When the HL7 transaction is received, first validation processor 25 parses the transaction message and compares the data in the transaction with the values specified in the configuration data in unit 37. If the data matches, indicating that the metadata is consistent with the trial protocol, first validation processor 25 passes the transaction message to research repository 43 in a format that the repository accepts. Integration engine 20 reformats the transaction message to be compatible with repository 43 as necessary. If any of the data checks fail, the message is routed to a holding queue and an alert message 33 is generated and routed to notify an appropriate person of a need for manual intervention. Alert message 33 indicates an image is unacceptable in response to unsuccessful first and second comparisons, for example.

Configuration information in unit 37 also indicates other mandatory criteria to be met by metadata 15 including data indicating, a data source (e.g., vendor or application name), a date an image was generated, a modality (e.g., MRI, CT scan, X-ray, Ultrasound) device that generated the image, a name of a clinician interpreting an image. Configuration information in unit 37 also indicates that some fields in the metadata cannot be blank such as an image assessment by an interpreting clinician.

Integration engine 20 accesses imaging information. Received image representative data 17 is transferred to a location in repository (or file server) 47. An image file is opened and the imaging metadata is copied into memory within integration engine 20 for processing. Configuration unit 37 contains predetermined information obtained from a clinical trial protocol indicating which body part images are contained, what modality devices are producing the images, how the image should be produced (e.g. slice thickness, percent sampling, etc.), and other criteria. The predetermined information is previously loaded into a database in unit 37 so that the requirements of a clinical trial image study processing protocol can be compared with information of the imaging metadata of an individual image. The database in configuration unit 37 also contains predetermined information describing how imaging metadata is parsed to allow integration engine 20 to obtain specific pieces of information from imaging metadata for comparison with requirements of the clinical trial image study protocol.

Second validation processor 30 in integration engine 20 uses the predetermined configuration information in unit 37 in parsing imaging metadata, examining the metadata and comparing them to the requirements of the clinical trial image study protocol. In the case of DICOM images the image header includes tags (0002,0000 through FFFE,E0DD) which provide information such as, but not limited to the following:
0008,0008 ImageType
0008,0060 Modality
0008,0050 AccessionNumber
0008,1030 Image Study Description
0018,0015 BodyPartExamined
0018,0050 Slice Thickness
0028,0004 Photometric Interpretation.

The data items acquired using the tags provide information on how the image was produced, the type of image and the body part contained in the image.

In an example of operation, an image file is opened and second validation processor 30 in integration engine 20 parses the image header using predetermined information in a database in configuration unit 37 derived from the clinical trial protocol to determine if the image is compatible with the protocol. Specifically, a clinical trial image study protocol for study XYZ specifies that for images to be used in a trial, a CT scan imaging Modality device is to be used, the Body Part to be imaged is the head, and Patient gender needs to be Female. Integration engine 20 derives this information from a clinical trial protocol and stores it in a database in unit 37 for use by second validation processor 30 in comparing the data items acquired using stored tags from the imaging metadata to determine if the image meets the requirements of a clinical trial image study protocol. Specifically, image representative data is received for patient 12345 including imaging metadata containing information related to the requirements of the clinical trial image study protocol. In the case of DICOM images the contained information and associated DICOM tags comprise, 0008,0008 ImageType; 0008,0060 Modality - CT; 0008,1030 Study Description - Study XYZ; 0010,0040 Patient Sex - Female; 0018,0015 BodyPartExamined - Head; 0018,0050 Slice Thickness - 6.000000 and 0028,0004 Photometric Interpretatio006.

Integration engine 20 compares data in image data header fields with required values stored in the configuration unit 37 database and if the header fields contain the required values, the image data is communicated to a system where it can be viewed by a clinician in detail. If the header does not contain the required values, engine 20 initiates an error processing procedure in processor 70. In response to a determination that image metadata or an image file cannot be processed, or a file is associated with an image that is not included in a clinical trial protocol, integration engine 20 either discards the information, or it routes the information to a holding queue and sends a notification that manual intervention is required. Error processor 70, in response to an unsuccessful first or second comparison, automatically initiates generation of alert message 33 to a user indicating the image is unacceptable and automatically routes image data to a storage location for further processing. Error processor 70 routes the image data to a storage location for manual processing via workstation 49, for example.

In an example, an image is received that does not comply with a clinical trial image study protocol. Specifically, during operation of a clinical trial, an image file is received for patient ABC as a result of a trip to an emergency room. The image is an MRI of the abdomen. Patient ABC is enrolled in trial XYZ. The XYZ trial protocol specifies use of CT scans of the head. Integration engine 20 parses the image header and determines that the image is not a CT scan of the head and discards the image file. Integration engine 20 is user configurable by setting data in a database in unit 37 to determine the type of error processing that occurs in response to detection of image data not complying with a clinical trial image study protocol.

In a further example, of error processing of image metadata, a metadata transaction message is received for patient 123 in trial DEF. Required information is found to be missing from the transaction message during the processing of the transaction. Integration engine 20 identifies that an error has occurred and terminates processing the transaction message data. The transaction message is routed within engine 20 to an error queue in error processor 70 and an alert message 33 is generated. Integration engine 20 routes a generated alert message via email, for example, to Investigator and Study Coordinator personnel to notify the personnel of the need for manual review and resolution.

Module 81 acquires automated image quality check data from units 25 and 30 and from engine 20 concerning the process that generated the quality check data. Integration engine 20 acquires data indicating, a source of an image (i.e., a trial site) as well as the content of metadata transactions and DICOM image headers. Engine 20 further acquires data identifying clinical sites providing digital (versus film) images, an imaging modality device that generated an image, modality device parameters used to generate an image, a digitization procedure used in generating an image, whether an initial quality of an image indicated it was rejected or accepted, and a reason an image failed an automated QC check. Additional information derived by engine 20 identifies sites that rely on film and only provide a metadata transaction describing an image (this may be indicated by a lack of supplied images for a trial, for example) and a percentage of images that pass automated quality checks versus those that fail for a given site or modality. For example, ten images are received from an image provider. Out of these ten, four images fail one or more quality checks (e.g., due to automatic detection of coding error, checksum error etc, and manual detection of image artefacts). The result is a sixty percent success rate for an image provider. In addition, engine 20 indicates an error rate per image for a site or modality. For example, site xyz provides 50 images over the course of a clinical trial. During a quality check of the images, a total of 17 errors are found in 11 images of the 50 provided. This yields an image error rate of one error per 2.9 images, calculated by dividing the number of images received by the number of errors found in those images.

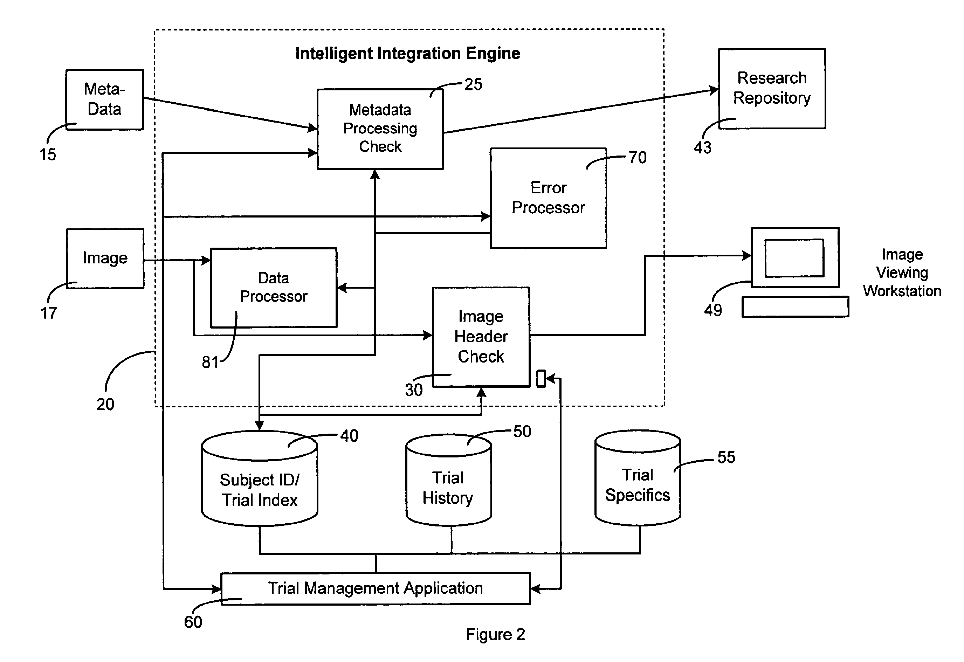

Figure 2 shows a further patient image data processing system embodiment. Integration engine 20 acquires and processes image meta-data by receiving data types and appropriately segmenting elements of the data involved in completing a quality control check. First validation processor 25 employs stored rule sets which comprise a clinical trial definition 55 to process data field values of incoming data to determine compliance with clinical trial requirements. First validation processor 25 also, as an initial step verifies patient and trial identification using a data element check. Image data elements within imaging metadata are extracted in appropriate sequence and validated against clinical trial criteria (an instance level check). Data indicating validation status is exchanged between first validation processor 25 and second validation processor 30 in support of validating image data against a clinical trial protocol. The validation status and image data is also compared with previous historical results 50 and context information 40 of the trial and patient to identify and validate change in status.

First validation processor 25 parses a message conveying patient medical image data to identify image metadata 15 indicating first characteristics of the image. The first characteristics of the image comprise, a data source identifier, a date an image was generated, data indicating a type of modality device and a user name. Processor 25 also performs a first comparison by comparing the metadata with configuration data indicating predetermined characteristics 55 of images required for a particular use (e.g., a clinical trial) and indicating the image is acceptable for the use in response to a successful first comparison. First validation processor 25 identifies image metadata indicating first characteristics of the image in a transaction message using transaction message data field identifiers. The transaction message and transaction message data field identifiers are HealthLevel7 protocol compatible, for example. First validation processor 25 compares the metadata with configuration data to determine at least one of, (a) an exact match with configuration data, (b) the metadata lies within a predetermined range and (c) required text is present in the metadata. First validation processor 25 compares the metadata with configuration data to determine the metadata is above or below a threshold value or to determine a comparison of the metadata with a value computed by the system.

Second validation processor 30 parses imaging metadata to identify image metadata 17 indicating second characteristics of the image. Processor 30 identifies image metadata indicating second characteristics of the image using - in the case of DICOM images - DICOM tags. The second characteristics of the image comprise, an image type identifier, a modality device identifier, an image study identifier, a modality imaging device setting or a patient characteristic. Processor 30 performs a second comparison by comparing header data with configuration data indicating predetermined characteristics 55 of images required for a particular use and indicating the image is acceptable for the use (e.g., a clinical trial) in response to a successful second comparison. Second validation processor 30 compares the header data with configuration data to determine at least one of, (a) an exact match with configuration data, (b) the header data is within a predetermined range and (c) required text is present in the header data. Processor 30 compares the header data with configuration data to determine the header data is above or below a threshold value or to determine a comparison of the header data with a value computed by the system.

Error processor 70, in response to an unsuccessful first or second comparison, automatically initiates generation of an alert message to a user indicating the image is unacceptable and automatically routes image data to a storage location for further processing. Error processor 70 routes the image data to a storage location for manual processing via workstation 49, for example. Data processor 81 indicates to trial management application 60 that the image is acceptable for the use in response to successful first and second comparisons and stores acceptable data in repository 43.

In response to image data failing a validation against criteria determined in a clinical trial definition performed by validation processors 25 and 30, integration engine 20 outputs messages to externally log validation check results and events together with a relevant time stamp, data element identifier, and data indicating a nature of the validation failure. In response to image data successfully passing validation by processors 25 and 30, integration engine 20 records the success status and passes the image data appropriately to a next module of the overall clinical trial system. The system advantageously provides fast and reliable data quality assessment checking compliance with a clinical trial protocol automatically without user interaction or with partial user interaction. The system is modular and advantageously extendable following needs of an individual clinical trial. The system reduces required time involved in monitoring clinical trial data and provides objective criteria for the quality assessment of clinical sites or data providers.

The systems of Figures 1 and 2 are not exclusive. Other systems, processes and menus may be derived in accordance with the principles of the invention to accomplish the same objectives. Although this invention has been described with reference to particular embodiments, it is to be understood that the embodiments and variations shown and described herein are for illustration purposes only. Modifications to the current design may be implemented by those skilled in the art, without departing from the scope of the invention. Further, any of the functions provided in the systems of Figures 1 and 2 may be implemented in hardware, software or a combination of both and may reside on one or more processing devices located at any location of a network linking system elements or another linked network including another intra-net or the Internet.

## Claims

1. A patient image data processing system, comprising:
a first validation processor for,
parsing a message conveying patient medical image data from an image provider to identify image metadata indicating first characteristics of said image and
performing a first comparison by comparing said metadata with configuration data indicating predetermined characteristics of images required for a particular use and providing first acceptability data indicating said image is unacceptable for said use in response to an unsuccessful first comparison;
a second validation processor for,
parsing imaging metadata representing said image to identify image metadata indicating second characteristics of said image and performing a second comparison by comparing header data with configuration data indicating predetermined characteristics of images required for a particular use and providing second acceptability data indicating said image is unacceptable for said use in response to an unsuccessful second comparison; and
a data processor for generating performance data using said first and second acceptability data indicating quality of images provided by said image provider.

2. A system according to claim 1, wherein
said imaging metadata comprises header data of DICOM compatible image representative data,
said image provider is a clinical trial participant and
said data processor generates said performance data comprising statistical information indicating relative proportion of acceptable and unacceptable images provided by said image provider using said first and second acceptability data and data indicating a number of acceptable images provided by said image provider, within a particular time duration.

3. A system according to claim 1, wherein
said configuration data indicates predetermined characteristics of images conforming to clinical trial requirements and
said data processor indicates said image is acceptable for use in a clinical trial in response to successful first and second comparisons.

4. A system according to claim 1, wherein
said data processor initiates generation of an alert message to a user indicating said image is unacceptable in response to unsuccessful first and second comparisons.

5. A system according to claim 1, including
an error processor for, in response to unsuccessful first and second comparisons,
initiating generation of an alert message to a user indicating said image is unacceptable and
routing image data to a storage location for further processing.

6. A system according to claim 5, wherein
said error processor routes said image data to a storage location for manual processing.

7. A system according to claim 1, wherein
said first validation processor compares said metadata with configuration data to determine at least one of, (a) an exact match with configuration data, (b) said metadata lies within a predetermined range and (c) required text is present in said metadata.

8. A system according to claim 1, wherein
said second validation processor compares said header data with configuration data to determine at least one of, (a) an exact match with configuration data, (b) said header data lies within a predetermined range and (c) required text is present in said header data.

9. A system according to claim 1, wherein
said first validation processor compares said metadata with configuration data to determine at least one of, (a) said metadata is above or below a threshold value and (b) a comparison of said metadata with a value computed by the system.

10. A system according to claim 1, wherein
said second validation processor compares said header data with configuration data to determine at least one of, (a) said header data is above or below a threshold value and (b) a comparison of said header data with a value computed by the system.

11. A patient image data processing system, comprising:
a first validation processor for automatically,
parsing a message conveying patient medical image data to identify image metadata indicating first characteristics of said image and
performing a first comparison by comparing said metadata with configuration data indicating predetermined characteristics of images required for a particular use and providing first acceptability data indicating said image is unacceptable for said use in response to an unsuccessful first comparison;
an error processor for, in response to an unsuccessful first comparison,
automatically initiating generation of an alert message to a user indicating said image is unacceptable and
automatically routing image data to a storage location for further processing; and
a data processor for generating performance data using said first acceptability data indicating quality of images provided by said image provider.

12. A system according to claim 11, wherein
said image provider is a clinical trial participant and
said data processor generates said performance data comprising statistical information indicating relative proportion of acceptable and unacceptable images provided by said image provider using said first acceptability data and data indicating a number of acceptable images provided by said image provider, within a particular time duration.

13. A patient image data processing system, comprising:
a first validation processor for automatically,
parsing imaging metadata representing said image to identify image metadata indicating second characteristics of said image and
performing a first comparison by comparing header data with configuration data indicating predetermined characteristics of images required for a particular use and providing first acceptability data indicating said image is unacceptable for said use in response to an unsuccessful first comparison;
an error processor for, in response to an unsuccessful first comparison,
automatically initiating generation of an alert message to a user indicating said image is unacceptable and
automatically routing image data to a storage location for further processing; and
a data processor for generating performance data using said first acceptability data indicating quality of images provided by said image provider.

14. A system according to claim 13, wherein
said image provider is a clinical trial participant and
said data processor generates said performance data comprising statistical information indicating relative proportion of acceptable and unacceptable images provided by said image provider using said first acceptability data and data indicating a number of acceptable images provided by said image provider, within a particular time duration.

15. A patient image data processing system, comprising:
a first validation processor for,
parsing messages conveying patient medical image data from a plurality of different image providers to identify image metadata indicating first characteristics of images and
performing a first comparison by comparing said metadata with configuration data indicating predetermined characteristics of images required for a particular use and providing first acceptability data indicating said images are unacceptable for said use in response to an unsuccessful first comparison;
a second validation processor for,
parsing imaging metadata representing said images to identify image metadata indicating second characteristics of said images and
performing a second comparison by comparing header data with configuration data indicating predetermined characteristics of images required for a particular use and providing second acceptability data indicating said images are unacceptable for said use in response to an unsuccessful second comparison; and
a data processor for generating performance data indicating relative performance of said plurality of different image providers using said first and second acceptability data indicating quality of images provided by said plurality of different image providers.
